# EUROPEAN PATENT APPLICATION

(11) **EP 4 206 190 A1**
(43) Date of publication of application: **05.07.2023**
(21) Application number: 20953772.9
(22) Date of filing: 21.09.2020
(51) Int. Cl.: C07D 291/06

(54) **REFINING METHOD FOR ACESULFAME**

(71) Applicant: Anhui Jinhe Industrial Co., Ltd., Chuzhou, Anhui 239200 (CN)
(72) Inventor: WANG, Congchun, Chuzhou, Anhui 239200 (CN); CHEN, Yongxu, Chuzhou, Anhui 239200 (CN); SHEN, Xiaofeng, Chuzhou, Anhui 239200 (CN); CHEN, Chaohui, Chuzhou, Anhui 239200 (CN); SHEN, Dongdong, Chuzhou, Anhui 239200 (CN)
(74) Representative: Schmid, Nils T.F.
(86) International application number: PCT/CN2020/116530
(87) International publication number: WO 2022/056914

(57) **Abstract**

Provided is a method for refining acesulfame potassium (Ace K), including: adding hydrogen peroxide and activated carbon to an Ace K crude product-containing solution to obtain a mixture, maintaining the mixture at a first preset temperature for a first preset time, and filtering the mixture to obtain an Ace K mother liquor; conducting concentration on the Ace K mother liquor to a preset concentration to obtain a concentrated solution, allowing the concentrated solution to stand at a second preset temperature for a second preset time to form an Ace K crystal nucleus, and obtaining a crystal nucleus-containing solution; conducting programmed freezing on the crystal nucleus-containing solution to obtain a solution with a large number of Ace K crystals; and conducting centrifugation, water washing, and drying on the solution with a large number of Ace K crystals to obtain an Ace K crystal product. The present disclosure has the following beneficial effects: by controlling crystallization process and technology, a high-quality and high-purity crystallization product is obtained with a less impurity content, and purity and quality of the Ace K crystal are significantly improved; moreover, the method for refining Ace K has a simple process, mild and controllable conditions, and low requirements for equipment and personnel skills, which is extremely suitable for large-scale industrial production.

## Description

### TECHNICAL FIELD

The present disclosure belongs to the field of fine chemical manufacturing, and in particular relates to a method for refining acesulfame potassium (Ace K).

### BACKGROUND

In the traditional Ace K production, a refining process includes: separating an organic phase to obtain a mother liquor, commonly known as syrup; subjecting the mother liquor to a series of steps such as decolorization, heating concentration, freezing crystallization, and centrifugal separation to obtain raw sugar (Yu Jiang, Synthesis Research of Sweetener Acesulfame-K, "Science and Technology Innovation Herald" 2008, 35, 9); and subjecting the raw sugar to recrystallization at least twice to obtain a qualified Ace K product. At present, the biggest difficulty during the production and refining of Ace K is that organic impurities produced in the production may be carried from an initial sugar to a final product, resulting in excessive organic impurities in the sugar and a relatively dark color of the product. Manufacturers have been plagued by product purity and quality problems. Therefore, the purification of Ace K determines the purity and quality of the final product. To trace the cause, one is that the purification and decolorization of organic impurities in the syrup cannot meet the requirements; and the other is that the crystallization is not controlled in place, making the purity, production efficiency, and yield of the product unsatisfactory, thereby further affecting a production cost of the product. These causes may lead to problems such as poor color of syrup, incomplete crystallization, high impurity content, limited purity, and low yield.

At present, the purification process from syrup to qualified products is intermittent, which has high energy consumption and serious waste of resources for multiple crystallization, cumbersome human operations, and high production costs, as well as complex and diverse reaction device, large temperature changes, and short service life of the equipment. Moreover, due to intermittent production, the utilization rate of the device is low, and the impurity content of the finished product is likely to exceed the standards. Accordingly, the product has a quality that cannot meet market requirements, seriously affecting its market value.

### SUMMARY

In view of the above, a method for refining Ace K is provided in the present disclosure to overcome the above problems or at least partially solve the above problems.

According to one aspect of the present disclosure, provided is a method for refining Ace K, including the following steps:
pretreatment: adding hydrogen peroxide and activated carbon to an Ace K crude product-containing solution to obtain a mixture, maintaining the mixture at a first preset temperature for a first preset time, and filtering the mixture to obtain an Ace K mother liquor;
nucleation: conducting concentration on the Ace K mother liquor to a preset concentration to obtain a concentrated solution, allowing the concentrated solution to stand at a second preset temperature for a second preset time to form an Ace K crystal nucleus, and obtaining a crystal nucleus-containing solution;
crystallization: conducting programmed freezing on the crystal nucleus-containing solution to obtain a solution with a large number of Ace K crystals; and
refining: conducting centrifugation, water washing, and drying on the solution with a large number of Ace K crystals to obtain an Ace K crystal product.

According to another aspect of the present disclosure, provided is an Ace K crystal prepared by the method for refining Ace K, where the Ace K crystal has a purity of greater than 99.20%, an organic impurity content of lower than 10 ppm, and a moisture content of lower than 0.3 wt%.

Some embodiments provide by the present disclosure have the following beneficial effects: by controlling the pretreatment, a solution to be crystallized (i.e., the syrup) is subjected to oxidative decolorization, which reduces the content of organic impurities in the initial sugar of Ace K and prevents the organic impurities from being brought into a final crystallization product, resulting in defects such as low purity and yellow color of the product. In addition, by controlling crystallization process and technology, a high-quality and high-purity crystallization product is obtained with a less impurity content, and purity and quality of the Ace K crystal are significantly improved. Moreover, the method for refining Ace K has a simple process, mild and controllable conditions, and low requirements for equipment and personnel skills, which is extremely suitable for large-scale industrial production.

The above description is merely a summary of the technical solutions of the present disclosure. In order to allow the technical means of the present disclosure to be understood clearly and implemented in accordance with the content of the specification and allow the above and other objects, features, and advantages of the present disclosure to be understood easily, specific embodiments of the present disclosure are described below.

### BRIEF DESCRIPTION OF THE DRAWINGS

By reading the detailed description of the following embodiments, various other advantages and benefits will become apparent to those of ordinary skill in the art. The accompanying drawing is provided merely to illustrate some embodiments, rather than to limit the present disclosure. Throughout the accompanying drawing, the same reference numerals represent the same component. In the accompanying drawing:

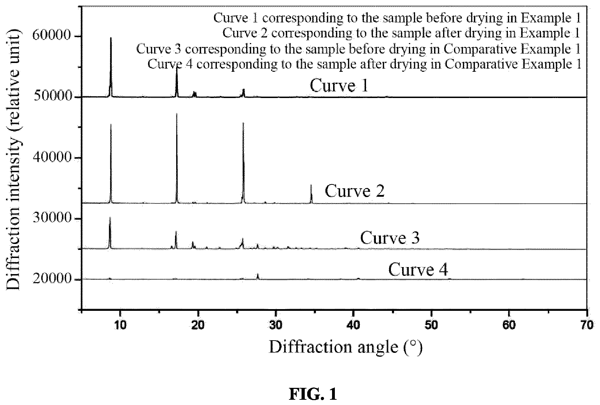

FIG. 1 shows an X-ray diffraction (XRD) pattern of Ace K crystals prepared according to Example 1 and Comparative Example 1.

### DETAILED DESCRIPTION OF THE EMBODIMENTS

Exemplary embodiments of the present disclosure are described in detail below with reference to the accompanying drawing. Although the accompanying drawing illustrates exemplary embodiments of the present disclosure, it should be understood that the present disclosure may be implemented in various forms and should not be limited to the embodiments set forth herein. Instead, these embodiments are provided to provide a thorough understanding of the present disclosure, and a scope of the present disclosure can be fully conveyed to those skilled in the art.

In the present disclosure, an concept lies in that: a solution to be crystallized (i.e. syrup) of Ace K is oxidized and decolorized through oxidation-reduction and physical adsorption, so as to reduce the content of organic impurities in the initial sugar; the initial sugar forms a crystal nucleus through concentration and heat preservation; and the crystal nucleus is fully crystallized through secondary nucleation, such that an Ace K crystal is obtained with a desirable crystal form and a high purity, thereby significantly reducing the impurity content of peritectic and improving a purity of the product.

In the present disclosure, a method for refining Ace K includes the steps as described below:
**Pretreatment:** adding hydrogen peroxide and activated carbon to an Ace K crude product-containing solution to obtain a mixture, maintaining the mixture at a first preset temperature for a first preset time, and filtering the mixture to obtain an Ace K mother liquor.

During the Ace K production, crystallization and purification, as highly important steps, are directly related to the quality of product. The Ace K crude product solution obtained in the Ace K production contains a large number of organic impurities, such as acetoacetamide, and further contains some colored impurities, such as compounds produced by dimerization of diketene, including diacetyl trisphenol and trimerized tetrafenpyrrole (E. Marcus and J. K. Chan, Novel Confensation Products of Diketene, J. Org. Chem. 1967, 32, 9, 2881-2887.). In the pretreatment, impurities such as acetoacetamide and multimers are treated with hydrogen peroxide. The hydrogen peroxide reacts with acetoacetamide and multimers in the organic impurities to undergo oxidation-reduction to generate hydrophilic carboxylic acids and the like. In the crystallization and refining, substances such as hydrophilic carboxylic acids remain in the solution and do not enter the final product.

In the present disclosure, some colored impurities are adsorbed by activated carbon, a type of specially-treated carbon. Heating organic raw materials (such as husks, coal, and wood) in the absence of air to reduce non-carbon components is called carbonization. A carbonization product reacts with the gas such that the surface of the carbonization product is eroded to produce a microporous structure, which is called activation. Activation is a microscopic process, that is, the surface erosion of a large number of molecular carbides is point erosion, resulting in countless fine pores on the surface of activated carbon. The surface micropores of activated carbon have diameters of mostly 2 nm to 50 nm. Even a small amount of activated carbon has a huge surface area, with a surface area of 500 m² to 1500 m² per gram of activated carbon. The huge surface area of activated carbon promotes adsorption of the colored impurities in the solution, thereby preventing the colored impurities from entering a final crystallization product, so as to improve purity and color grade of the product.

**Nucleation:** conducting concentration on the Ace K mother liquor to a preset concentration to obtain a concentrated solution, allowing the concentrated solution to stand at a second preset temperature for a second preset time to form an Ace K crystal nucleus, and obtaining a crystal nucleus-containing solution.

**Crystallization:** conducting programmed freezing on the crystal nucleus-containing solution to obtain a solution with a large number of Ace K crystals.

In the present disclosure, in the method for refining Ace K, a process for forming crystals includes two steps, one corresponding to the nucleation and the other corresponding to the crystallization. The nucleation is to form a small amount of crystal nuclei in the Ace K mother liquor by controlling conditions; these nuclei act as seed crystal during the crystallization, providing a basis for the massive growth of crystals.

In the prior art, Ace K crystals are generally obtained through repeated recrystallization. There are many defects in the crystals generated in this way, such as low purity of the product, low yield, complex process, and high requirements for equipment and personnel skills. However, in the present disclosure, the existing crystallization is divided into two steps, where the formation of crystal nuclei of Ace K is promoted through the control of conditions; and based on the formed crystal nuclei, the rapid formation of a large number of Ace K crystals is promoted by means of programmed freezing.

**Refining:** conducting centrifugation, water washing, and drying on the solution with a large amount of Ace K crystals to obtain an Ace K crystal product.

Finally, a high-purity and high-yield Ace K crystal product can be obtained through conventional post-treatment techniques, including but not limited to centrifugation, water washing, and drying.

In the present disclosure, by controlling the pretreatment, a solution to be crystallized (i.e., the syrup) is subjected to oxidative decolorization, which reduces a content of organic impurities in the initial sugar of Ace K and prevents the organic impurities from being brought into a final crystallization product, resulting in defects such as low purity and yellow color of the product. In addition, by controlling the crystallization process and technology, a high-quality and high-purity crystallization product is obtained with a less impurity content, and purity and quality of the Ace K crystal are significantly improved. Moreover, the method for refining Ace K has a simple process, mild and controllable conditions, and low requirements for equipment and personnel skills, which is extremely suitable for large-scale industrial production.

### Amount of hydrogen peroxide

In the present disclosure, there is no limitation on the amount of the hydrogen peroxide; in some embodiments, the hydrogen peroxide is present in an amount of 0.1 wt% to 10 wt%, preferably 0.5 wt% to 2 wt% of a total mass of the Ace K crude product-containing solution. Since the amount of organic impurities in the Ace K crude product-containing solution is predictable or estimable, the dosage of hydrogen peroxide can be determined according to a total mass of the Ace K crude product-containing solution. If the amount of hydrogen peroxide is less than 0.1 wt% of the total mass of the Ace K crude product-containing solution, hydrogen peroxide has an insufficient amount, and thus cannot completely neutralize the reducing organic impurities. If the amount of hydrogen peroxide is more than 10 wt% of the total mass of the Ace K crude product-containing solution, hydrogen peroxide has an excessive amount, and the excess hydrogen peroxide may corrode equipment due to strong oxidizing properties, making the post-treatment complicated and difficult to handle.

### Amount of activated carbon

In the present disclosure, there is no limitation on the amount of the activated carbon; in some embodiments, the activated carbon is in an amount of 0.1 wt% to 5 wt%, preferably 0.2 wt% to 4 wt% of a total mass of the Ace K crude product-containing solution. Since the amount of colored impurities in the Ace K crude product-containing solution is predictable or estimable, the dosage of activated carbon can be determined according to a total mass of the Ace K crude product-containing solution. If the amount of activated carbon is less than 0.1 wt% of the total mass of the Ace K crude product-containing solution, the activated carbon has an insufficient amount, and thus cannot completely absorb the colored impurities in the Ace K crude product-containing solution. If the amount of activated carbon is more than 5 wt% of the total mass of the Ace K crude product-containing solution, the amount is excessive, causing unnecessary waste, and increasing the burden on subsequent filtering procedures.

### Reaction conditions in the pretreatment

In the pretreatment, two reactions occur simultaneously: redox chemical reaction between hydrogen peroxide and organic impurities, and the physical adsorption of activated carbon on colored impurities. The two reactions can only achieve a desired effect under certain conditions. Through a lot of screening, it is determined that in the pretreatment, the first preset temperature is in the range of 40°C to 90°C; and the first preset time is in the range of 1 h to 8 h.

If the first preset temperature is less than 40°C and the first preset time is less than 1 h, the redox chemical reaction and physical adsorption cannot be completed due to too mild conditions and too short contact time. If the first preset temperature is greater than 90°C and the first preset time is greater than 8 h, the redox chemical reaction and physical adsorption are too sharp due to too intense conditions and too long contact time; especially, the redox chemical reaction may even cause localized bumping.

### Concentration methods and conditions in the nucleation

In the present disclosure, during the nucleation, there is no limitation on the methods and conditions of the concentration. In some embodiments, the concentration is conducted by vacuum distillation at a temperature of 45°C to 80°C, preferably 55°C to 65°C and at a pressure of -95 KPa to -101 KPa for 10 min to 12 min. The conditions for the vacuum distillation are to quickly make the Ace K mother liquor reach the preset concentration without causing excessive load to the equipment.

### Preset concentration in the nucleation

In the present disclosure, during the nucleation, there is no limitation on a preset concentration. In some embodiments, the preset concentration is that a solid phase accounts for 30 wt% to 90 wt%, preferably 40 wt% to 80 wt% of a total mass of the Ace K mother liquor.

The preset concentration may be related to whether subsequent crystal nuclei and crystals can be successfully generated. During the concentration, if the content of the solid phase in the Ace K mother liquor is less than 30 wt% of a total mass of the Ace K mother liquor, there may be too much solvent, making it difficult to generate crystal nuclei in the subsequent nucleation. If the content of the solid phase in the Ace K mother liquor is greater than 90 wt% of the total mass of the Ace K mother liquor, there may be too little solvent, resulting in rapid evaporation of the solvent during the subsequent heat preservation of the nucleation, and the formed crystal nuclei and crystals cannot be effectively separated. As a result, a large number of irregular crystals are generated, which may even lead to failure of the entire refining.

### Temperature and time in the nucleation

In the present disclosure, during the nucleation, the second preset temperature is in the range of 40°C to 70°C, preferably 45°C to 60°C; and the second preset time is in the range of 0.1 h to 4 h, preferably 0.5 h to 2 h.

During the nucleation, the temperature and time for heat preservation may be important parameters to promote the nucleation. If these parameters are not properly controlled, crystal nuclei cannot be generated. In some embodiments, the second preset temperature is in the range of 40°C to 70°C, and the second preset time is in the range of 0.1 h to 4 h; and in some other embodiments, the preset temperature is in the range of 45°C to 60°C, and the second preset time is in the range of 0.5 h to 2 h. If the second preset temperature is less than 40°C, the temperature is too low, which may cause the solvent to evaporate too slowly during the nucleation, making it difficult to precipitate crystal nuclei. If the second preset temperature is greater than 60°C, the temperature is too high, which may result in extremely irregular crystal nuclei being precipitated during the nucleation. The time also requires a lot of investigation. If the second preset time is shorter than 0.1 h, the time is too short, which may cause the crystal nuclei to not yet precipitate or the crystal nuclei to just start to precipitate or only a small amount of crystal nuclei to precipitate; too few crystal nuclei cannot provide an effective seed crystal for subsequent crystallization. If the second preset time is longer than 2 h, the time is too long, which may cause a large amount of crystal nuclei precipitated; even some crystals with complicated crystal forms are produced, which are not the crystal forms expected in the present disclosure.

### Programmed freezing conditions in the crystallization

In the present disclosure, during the crystallization, the programmed freezing conditions may be a key directly related to whether a large amount of high-quality crystals can be formed quickly; in some embodiments, the programmed freezing includes: cooling down from the second preset temperature to an intermediate temperature of 40°C to 45°C at a rate of 1°C/10 min to 1°C/60 min and holding the intermediate temperature for 0.5 h to 8 h, and then cooling down from the intermediate temperature to 8°C to 12°C at a rate of 1°C/10 min to 1°C/60 min. In some other embodiments, the programmed freezing includes: cooling down from the second preset temperature to an intermediate temperature of 45°C at a rate of 1°C/10 min to 1°C/60 min and holding the intermediate temperature for 0.5 h to 8 h, and cooling down from the intermediate temperature to 10°C at a rate of 1°C/10 min to 1°C/60 min.

A high-purity Ace K crystal can be obtained by any one of the above methods. The Ace K crystal has a purity of higher than 99.30%, an organic impurity content of lower than 10 ppm, and a moisture content of lower than 0.3 wt%.

In the prior art, Ace K in an existing Ace K product generally has a purity of 93.0 wt% to 99.0 wt%, and it is difficult to achieve a purity of more than 99.0%. The Ace K product prepared by the refining method provided in the present disclosure has a purity of greater than 99.0%, an organic impurity content of lower than 10 ppm, and a moisture content of lower than 0.3 wt%.

The Ace K crystals prepared according to some embodiments in the present disclosure are measured by XRD. Before drying, the Ace K crystal has three strongest diffraction peaks at 8.8±0.2, 17.3±0.2, and 28.8±0.2, respectively; and after drying, the Ace K crystal has an XRD spectrum peak intensity weaker than that of the Ace K crystal before drying, showing a strongest diffraction peak at 8.8±0.2 and a second strongest diffraction peak at 17.3±0.2. It can be seen that the prepared wet Ace K sample has a desirable crystal structure, showing simple and strong XRD peaks; after the sample is dried to obtain an anhydrous sample, a part of the crystal structure is destroyed, and the XRD peaks of the sample are also weaker than those before drying.

### Test method

In the present disclosure, the test methods adopted in each example and comparative example are as follows, and are not repeated in each example.

In the present disclosure, the methods for analysis and test of Ace K could refer to the national standard of food additives GB/T5009.140-2003, *"Determination of acesulfame K (Ace K) in beverages".*

Conditions for the analysis and test of Ace K: high-performance liquid chromatograph of Shimadzu, Japan (ultraviolet detector), LC-10ADVP high-pressure pump, CTO-10ASVP constant-temperature box; chromatographic column: Agilent XDB C18 column (250 mm×4.6 mm, 5 µm); mobile phase: 0.02 mol/L ammonium sulfate (780 mL) + methanol (100 mL) + acetonitrile (30 mL); column temperature: 30°C; and flow rate: 0.8 mL/min. The content was measured by an external standard method.
Method or conditions for determination of impurity content (residue on ignition): the same as above.
Measurement conditions of XRD: an X-ray diffractometer, with a model of Broker B8 Advance, using Kα1 ray of Cu target, at a wavelength of 1.5418 nm.
Method and conditions for determination of moisture content: referring to the national standard GB/T 6283-2008 *"Chemical products* - *Determination of water*, *Karl.fischer method (general method)".*

### Example 1

0.5 g of activated carbon and 0.5 g of hydrogen peroxide were added to 200 mL of a to-be-crystallized Ace K solution (i.e., syrup, containing 20 wt % of Ace K) obtained in a previous process to obtain a mixture, the mixture was stirred and subjected to heat preservation at 80°C for 2 h, and then the activated carbon was then removed by filtration while the mixture was hot. A resulting syrup solution was concentrated by vacuum distillation at -0.095 MPa and 70°C for 1 h to a preset concentration of 30 wt%. A resulting concentrated solution was subjected to heat preservation at 60°C for 1 h to form an effective crystal nucleus. A resulting crystal nucleus-containing solution was cooled to an intermediate temperature of about 45°C at 1°C/20 min and subjected to heat preservation at the intermediate temperature for 2 h, and then cooled to about 10°C at 1°C/20 min to obtain a large number of crystals. A resulting solution containing the crystals was centrifuged and washed with a small amount of cold ultrapure water to obtain a wet centrifuged sample, and then dried to obtain 25 g of a high-purity solid crystal with a purity of 99.5%, an impurity content of 10 ppm, and a moisture content of 0.2 wt%. FIG. 1 shows an XRD pattern of the samples measured before and after drying.

### Comparative Example 1

200 mL of a to-be-crystallized Ace K solution (i.e., syrup, containing 20 wt % of Ace K) obtained in a previous process was directly concentrated by vacuum distillation at -0.095 MPa and 70°C for 1 h until a solid concentration of the Ace K was 30 wt% to obtain an Ace K mother liquor, and the Ace K mother liquor was rapidly cooled to 10°C, and crystallized for 12 h to obtain a large amount of crystals. A resulting solution containing the crystals was centrifuged and washed with a small amount of cold ultrapure water to obtain a wet centrifuged sample, and then dried to obtain 21 g of a high-purity solid crystal with a purity of 99.0%, an impurity content of 50 ppm, and a moisture content of 0.3 wt%. Fig. 1 shows an XRD pattern of the samples measured before and after drying.

The results of Example 1 and Comparative Example 1 show that 25 g of an Ace K solid was prepared according to Example 1, and 21 g of an Ace K solid was prepared according to Comparative Example 1, indicating that the yield of Ace K crystals of Example 1 is far greater than that of Comparative Example 1.

Fig. 1 shows the XRD pattern of Ace K crystals prepared according to Example 1 and Comparative Example 1. Curves 1 and 3 show the XRD pattern of the Ace K samples prepared according to Example 1 and Comparative Example 1 before drying, respectively, i.e. the XRD pattern of the wet samples; and curves 2 and 4 show the XRD pattern of the Ace K samples prepared according to Example 1 and Comparative Example 1 after drying, respectively, i.e. the XRD pattern of the dry samples. As shown in Fig. 1, the crystals obtained by the two methods according to Example 1 and Comparative Example 1 have extremely large differences in structure:

First, it can be seen from curve 3 in Fig. 1 that the Ace K sample prepared according to Comparative Example 1 has almost no signal before drying, indicating that the sample was basically an amorphous structure. However, as shown in curve 1, there is a high XRD spectrum peak intensity of the Ace K sample before drying prepared according to Example 1; the peak is simple while the intensity is high, indicating an excellent crystal structure. The corresponding three strongest diffraction peaks are at 8.8±0.2, 17.3±0.2, and 28.8±0.2, respectively, and there is a small peak at 34.5±0.2.

Second, it can be seen from curves 2 and 4 that the samples obtained according to Comparative Example 1 and Example 1 have obvious changes in the crystals after drying to obtain anhydrous samples. The dried Ace K sample prepared according to Comparative Example 1 form a certain crystal structure by the drying process, with a main spectral peak position almost consistent with that of the dried sample prepared by the method provided in the present disclosure, indicating that the main crystal forms are basically the same. However, the spectrum peak of Ace K prepared according to Comparative Example 1 after drying is weaker than that of the method provided in the present disclosure, which also shows that the final product according to Example 1 has a better crystal structure. Comparing the XRD spectrum of the sample prepared according to Example 1 provided in the present disclosure after drying, it can be found that the spectral peaks have obvious changes before and after drying. The strongest diffraction peak of the sample after drying is at 8.8±0.2, the next strongest peak is at 17.3±0.2, there is a small peak at 28.8±0.2, the small peak at 34.5±0.2 disappears, and small peaks appear at 19.5±0.2 and 28.6±0.2. This shows that a part of the crystal structure is destroyed during the drying, and the crystal form integrity of the sample decreases after the product is dried, and crystal forms involving other structures appear.

### Example 2

0.5 g of activated carbon and 0.5 g of hydrogen peroxide were added to 200 mL of a to-be-crystallized Ace K solution (i.e., syrup, containing 20 wt % of Ace K) obtained in a previous process to obtain a mixture, the mixture was stirred and subjected to heat preservation at 80°C for 2 h, and the activated carbon was then removed by filtration while the mixture was hot. A resulting syrup solution was concentrated by vacuum distillation at -0.095 MPa and 70°C for 1 h to a preset concentration of 40 wt%. A resulting concentrated solution was subjected to heat preservation at 60°C for 1 h to form an effective crystal nucleus. A resulting crystal nucleus-containing solution was cooled to an intermediate temperature of about 45°C at 1°C/10 min and subjected to heat preservation at the intermediate temperature for 1 h, and then cooled to about 10°C at 1°C/10 min to obtain a large number of crystals. A resulting solution containing the crystal was centrifuged and washed with a small amount of cold ultrapure water to obtain a wet centrifuged sample, and then dried to obtain 31 g of a high-purity solid crystal a purity of 99.2%, an impurity content of 20 ppm, and a moisture content of 0.3 wt%. Fig. 1 shows an XRD pattern of the samples measured before and after drying.

### Example 3

0.5 g of activated carbon and 0.5 g of hydrogen peroxide were added to 200 mL of a to-be-crystallized Ace K solution (i.e., syrup, containing 20 wt % of Ace K) obtained by a previous process to obtain a mixture, the mixture was stirred and subjected to heat preservation at 80°C for 2 h, and the activated carbon was then removed by filtration while the mixture was hot. A resulting syrup solution was concentrated by vacuum distillation at -0.095 MPa and 70°C for 1 h to a preset concentration of 45 wt%. A resulting concentrated solution was subjected to heat preservation at 50°C for 1 h to form an effective crystal nucleus. A resulting crystal nucleus-containing solution was cooled to an intermediate temperature of about 40°C at 1°C/30 min and subjected to heat preservation at the intermediate temperature for 2 h, and then cooled to about 10°C at 1°C/30 min to obtain a large number of crystals. A resulting solution containing the crystals was centrifuged and washed with a small amount of cold ultrapure water to obtain a wet centrifuged sample, and then dried to obtain 32g of a high-purity solid crystal a purity of 99.6%, an impurity content of 4 ppm, and a moisture content of 0.2 wt%.

### Example 4

0.5 g of activated carbon and 0.5 g of hydrogen peroxide were added to 200 mL of a to-be-crystallized Ace K solution (i.e., syrup, containing 25 wt % of Ace K) obtained in a previous process to obtain a mixture, the mixture was stirred and subjected to heat preservation at 70°C for 2 h, and the activated carbon was then removed by filtration while the mixture was hot. A resulting syrup solution was concentrated by vacuum distillation at -0.095 MPa and 70°C for 1 h to a preset concentration of 45 wt%. A resulting concentrated solution was subjected to heat preservation at 50°C for 1 h to form an effective crystal nucleus. A resulting crystal nucleus-containing solution was cooled to an intermediate temperature of about 40°C at 1°C/30 min and subjected to heat preservation at the intermediate temperature for 2 h, and then cooled to about 10°C at 1°C/30 min to obtain a large number of crystals. A resulting solution containing the crystals was centrifuged and washed with a small amount of cold ultrapure water to obtain a wet centrifuged sample, and then dried to obtain 40g of a high-purity solid crystal a purity of 99.5%, an impurity content of 6 ppm, and a moisture content of 0.2 wt%.

### Example 5

0.5 g of activated carbon and 0.5 g of hydrogen peroxide were added to 200 mL of a to-be-crystallized Ace K solution (i.e., syrup, containing 25 wt % of Ace K) obtained in a previous process to obtain a mixture, the mixture was stirred and subjected to heat preservation at 80°C for 2 h, and the activated carbon was then removed by filtration while the mixture was hot. A resulting syrup solution was concentrated by vacuum distillation at -0.095 MPa and 70°C for 1 h to a preset concentration of 45 wt%. A resulting concentrated solution was subjected to heat preservation at 50°C for 1 h to form an effective crystal nucleus. A resulting crystal nucleus-containing solution was cooled to an intermediate temperature of about 40°C at 1°C/30 min and subjected to heat preservation at the intermediate temperature for 2 h, and then cooled to about 10°C at 1°C/30 min to obtain a large number of crystals. A resulting solution containing the crystals was centrifuged and washed with a small amount of cold ultrapure water to obtain a wet centrifuged sample, and then dried to obtain 42 g of a high-purity solid crystal a purity of 99.2%, an impurity content of 15 ppm, and a moisture content of 0.2 wt%.

### Comparative Example 2

200 mL of a to-be-crystallized Ace K solution (i.e., syrup, containing 25 wt % of Ace K) obtained in a previous process was heated and distilled, 0.5 g of activated carbon was added, stirred and subjected to heat preservation at 70°C for 2 h, and the activated carbon was removed by filtration while the mixture was hot. The treated resulting syrup solution was concentrated by vacuum distillation (-0.095 MPa, 70°C) for 1 h to a preset concentration of 40 wt%. A resulting concentrated solution was subjected to heat preservation at 55°C for 1 h to form an effective crystal nucleus. A resulting crystal nucleus-containing solution was cooled to an intermediate temperature of about 45°C at 1°C/10 min and subjected to heat preservation for 1 h at the intermediate temperature, and then cooled to about 10°C at 1°C/10 min to obtain a large amount of crystals. A resulting solution containing the crystals was centrifuged and washed with a small amount of cold ultrapure water to obtain a wet centrifuged sample, and then dried to obtain 38 g of a high-purity solid crystal a purity of 99.2%, an impurity content of 35 ppm, and a moisture content of 0.3 wt%.

### Comparative Example 3

200 mL of a to-be-crystallized Ace K solution (i.e., syrup, containing 25 wt % of Ace K) obtained in a previous process was heated and distilled, 0.5 g of hydrogen peroxide was added, stirred and subjected to heat preservation at 80°C for 2 h, and concentrated by vacuum distillation (-0.095 MPa, 70°C) for 1 h to a preset concentration of 40 wt%. A resulting concentrated solution was subjected to heat preservation at 60°C for 1 h to form an effective crystal nucleus. A resulting crystal nucleus-containing solution was cooled to an intermediate temperature of about 45°C at 1°C/10 min and subjected to heat preservation for 1 h at the intermediate temperature, and then cooled to about 10°C at 1°C/10 min to obtain a large amount of crystals. A resulting solution containing the crystals was centrifuged and washed with a small amount of cold ultrapure water to obtain a wet centrifuged sample, and then dried to obtain 37 g of a high-purity solid crystal a purity of 99.2%, an impurity content of 25 ppm, and a moisture content of 0.3 wt%.

In order to more intuitively compare the technical effects of the examples and the comparative examples, the properties and yields of the products obtained according to Examples 1 to 5 and Comparative Examples 1 to 3 are listed in Table 1. The solid content of Ace K in the solution to be crystallized is obtained by multiplying a volume of the to-be-crystallized Ace K solution by the corresponding mass fraction thereof.

**Table 1 Properties and yields of products obtained according to Examples 1 to 5 and Comparative Examples 1 to 3 (E referring to Example and CE referring to Comparative Example)**

| | The content of Ace K solid in the solution to be crystallized (g) | The mass of high-purity Ace K solid obtained after drying (g) | Calculated yield of Ace K (%) | Product purity(%, by HPLC) | Impurity content(ppm ) | Moisture content in Ace K product (w%) |
|---|---|---|---|---|---|---|
| E1 | 40 | 25 | 62.5 | 99.5 | 10 | 0.2 |
| E2 | 40 | 31 | 77.5 | 99.2 | 20 | 0.3 |
| E3 | 40 | 32 | 80 | 99.6 | 4 | 0.2 |
| E4 | 50 | 40 | 80 | 99.5 | 6 | 0.2 |
| E5 | 50 | 42 | 84 | 99.2 | 15 | 0.2 |
| CE1 | 40 | 21 | 52.5 | 99.0 | 50 | 0.3 |
| CE2 | 50 | 38 | 76 | 99.2 | 35 | 0.3 |
| CE3 | 50 | 37 | 74 | 99.2 | 25 | 0.3 |

In Comparative Example 2 and Comparative Example 3, the to-be-crystallized Ace K solution was pretreated by a single substance. As shown in the results of Example 5 and Comparative Examples 2-3, the final products according to Comparative Examples 2-3 have a higher impurity content than that of the final product obtained by using both hydrogen peroxide and activated carbon to simultaneously pretreat the to-be-crystallized Ace K solution. From Example 2 and Comparative Examples 2-3, it can be seen that the yield of Ace K crystals in Example 2 is significantly higher than that of Comparative Examples 2-3, indicating that the yield of the final product obtained by pretreatment of the to-be-crystallized Ace K solution with both hydrogen peroxide and activated carbon is higher than that of the final product obtained by pretreatment of the to-be-crystallized Ace K solution by a single substance. In addition, the refining effects of Example 1 and Comparative Example 1 are obviously not as good as that of Examples 2 to 5. This is because that the Ace K mother liquor was concentrated to a preset concentration of 30 wt% in Example 1 and Comparative Example 1, which had a refining effect not as good as that of the Ace K mother liquor concentrated to a preset concentration of 40 wt% or 45 wt%.

In summary, in the present disclosure, by controlling the pretreatment, a solution to be crystallized (i.e., the syrup) is subjected to oxidative decolorization, which reduces a content of organic impurities in initial sugar of Ace K and prevents the organic impurities from being brought into a final crystallization product, resulting in defects such as low purity and yellow color of the product; in addition, by controlling crystallization process and technology, a high-quality and high-purity crystallization product is obtained with a less impurity content, and purity and quality of the Ace K crystal are significantly improved; moreover, the method for refining Ace K has a simple process, mild and controllable conditions, and low requirements for equipment and personnel skills, which is extremely suitable for large-scale industrial production.

The above are merely specific embodiments of the present disclosure, and under the above instruction of the present disclosure, other improvements or variations on the basis of the above examples can be made by those skilled in the art. Those skilled in the art should understand that the above specific description is merely intended to well explain the purpose of the present disclosure, and a protection scope of the present disclosure shall be subject to the protection scope of the claims.

In addition, those skilled in the art should understand that, although some embodiments herein include some features included in other embodiments but not other features, a combination of features of different embodiments falls within the scope of the present disclosure and forms a different embodiment. For example, in the claims, any one of the claimed embodiments can be used in any combination.

## Claims

1. A method for refining acesulfame potassium (Ace K), comprising the following steps:
pretreatment: adding hydrogen peroxide and activated carbon to an Ace K crude product-containing solution to obtain a mixture, maintaining the mixture at a first preset temperature for a first preset time, and filtering the mixture to obtain an Ace K mother liquor;
nucleation: conducting concentration on the Ace K mother liquor to a preset concentration to obtain a concentrated solution, allowing the concentrated solution to stand at a second preset temperature for a second preset time to form an Ace K crystal nucleus, and obtaining a crystal nucleus-containing solution;
crystallization: conducting programmed freezing on the crystal nucleus-containing solution to obtain a solution with a large number of Ace K crystals; and
refining: conducting centrifugation, water washing, and drying on the solution with a large number of Ace K crystals to obtain an Ace K crystal product.

2. The method according to claim 1, wherein the hydrogen peroxide is used in an amount of 0.1 wt% to 10 wt%, preferably 0.5 wt% to 2 wt% of a total mass of the Ace K crude product-containing solution; and
the activated carbon is used in an amount of 0.1 wt% to 5 wt%, preferably 0.2 wt% to 4 wt% of the total mass of the Ace K crude product-containing solution.

3. The method according to claim 1, wherein in the pretreatment, the first preset temperature is in the range of 40°C to 90°C; and the first preset time is in the range of 1 h to 8 h.

4. The method according to claim 1, wherein in the nucleation, the concentration is conducted by vacuum distillation at a temperature of 45°C to 80°C, preferably 55°C to 65°C and a pressure of -95 KPa to -101 KPa for 10 min to 12 min.

5. The method according to claim 1, wherein in the nucleation, the preset concentration is that a solid phase accounts for 30 wt% to 90 wt%, preferably 40 wt% to 80 wt% of a total mass of the Ace K mother liquor.

6. The method according to claim 1, wherein in the nucleation, the second preset temperature is in the range of 40°C to 70°C, preferably 45°C to 60°C; and
the second preset time is in the range of 0.1 h to 4 h, preferably 0.5 h to 2 h.

7. The method according to claim 1, wherein in the crystallization, the programmed freezing comprises: cooling down from the second preset temperature to an intermediate temperature of 43°C to 45°C at a rate of 1°C/10 min to 1°C/60 min, holding the intermediate temperature for 0.5 h to 8 h, and cooling down from the intermediate temperature to 8°C to 12°C at a rate of 1°C/10 min to 1°C/60 min.

8. The method according to claim 7, wherein in the crystallization, the programmed freezing comprises: cooling down from the second preset temperature to the intermediate temperature of 45°C at a rate of 1°C/10 min to 1°C/60 min, holding the intermediate temperature for 0.5 h to 8 h, and cooling down from the intermediate temperature to 10°C at a rate of 1°C/10 min to 1°C/60 min.

9. An Ace K crystal, prepared by the method for refining Ace K according to any one of claims 1 to 8, wherein the Ace K crystal has a purity of greater than 99.20%, an organic impurity content of lower than 10 ppm, and a moisture content of lower than 0.3 wt%.

10. The Ace K crystal according to claim 9, wherein before drying, the Ace K crystal has three strongest diffraction peaks at 8.8±0.2, 17.3±0.2, and 28.8±0.2, respectively; and
after drying, the Ace K crystal has an X-ray diffraction (XRD) spectrum peak intensity weaker than that of the Ace K crystal before drying, showing a strongest diffraction peak at 8.8±0.2 and a second strongest diffraction peak at 17.3±0.2.
